# EUROPEAN PATENT APPLICATION

(11) **EP 2 412 786 A1**
(43) Date of publication of application: **01.02.2012**
(21) Application number: 10755701.9
(22) Date of filing: 26.03.2010
(51) Int. Cl.: C10G 35/14, B01J 29/87

(54) **METHOD FOR PRODUCING AROMATIC HYDROCARBON**

(30) Priority: 27.03.2009 JP 2009078602
(71) Applicant: Chiyoda Corporation, Kanagawa 230-8601 (JP); JX Nippon Oil & Energy Corporation, Chiyoda-ku Tokyo 100-0004 (JP)
(72) Inventor: MINAMI, Hideki, Yokohama-shi Kanagawa 230-8601 (JP); SUGI, Yoshishige, Yokohama-shi Kanagawa 230-8601 (JP); FUKUI, Atsushi, Yokohama-shi Kanagawa 230-8601 (JP); NAGUMO, Atsuro, Yokohama-shi Kanagawa 230-8601 (JP); YANAGAWA, Shinichiro, Chiyoda-ku Tokyo 1008162 (JP); HAYASAKA, Kazuaki, Yokohama-shi Kanagawa 231-0815 (JP)
(74) Representative: Vossius & Partner
(86) International application number: PCT/JP2010/002172
(87) International publication number: WO 2010/109899

(57) **Abstract**

A method for producing aromatic hydrocarbons in which at least one feedstock oil selected from the group consisting of LCO produced from an FCC apparatus, hydrotreated LCO, naphtha and straight-run gas oil is brought into contact with a reforming catalyst inside a fluidized bed reactor, wherein the method includes transporting a reforming catalyst that has been extracted from the fluidized bed reactor to a heating tank, heating the reforming catalyst in the heating tank to a temperature at least as high as the reaction temperature inside the fluidized bed reactor, and following heating, transporting the heated reforming catalyst to the fluidized bed reactor.

## Description

### TECHNICAL FIELD

The present invention relates to a method for producing aromatic hydrocarbons. The method relates particularly to a method for producing aromatic hydrocarbons by a catalytic reforming reaction using a fluidized bed reactor.
Priority is claimed on Japanese Patent Application No. 2009-078602, filed March 27, 2009, the content of which is incorporated herein by reference.

### BACKGROUND ART

Methods for producing aromatic hydrocarbons such as BTX (benzene, toluene, xylene and the like) by catalytic reforming of a light naphtha or heavy naphtha or the like obtained from a fluid catalytic cracking (hereinafter abbreviated as FCC) apparatus are well known. These production methods generally employ a system based on a fixed bed or moving bed that uses a granulated reforming catalyst.
Because the reforming reaction is usually an endothermic reaction, the method used for supplying the heat required by the reaction and the technique used for controlling the associated temperature are often problematic issues. Further, the generation of coke that accompanies the reaction operation, which is an issue even when a light naphtha is used as the feedstock, becomes far more marked when a heavy naphtha or particularly a light cycle oil (hereinafter abbreviated as LCO) or gas oil is used as the feedstock, and the frequency with which the reforming catalyst must be regenerated also increases markedly.

In order to address these issues, systems that use a fluidized bed catalytic reforming method have been proposed instead of the conventional fixed bed and moving bed systems (for example, see Patent Document 1).
By employing a fluidized bed system, the reforming catalyst of the reaction bed and the feedstock can be maintained in a state that is close to complete mixing, and therefore maintaining the reaction temperature at a constant level is simplified. At the same time, in the case of a heavier feedstock, a reforming catalyst that has been degraded with coke can be easily extracted from the fluidized bed reactor, enabling catalyst regeneration to be performed smoothly. In other words, when coke adheres to the reforming catalyst due to contact with the feedstock, the reforming catalyst becomes inactivated, and therefore the reforming catalyst is extracted from the fluidized bed reactor and transported to a regenerator, and the coke adhered to the reforming catalyst is combusted inside the regenerator to regenerate the reforming catalyst. The regenerated reforming catalyst is then transported back to the fluidized bed reactor.

However, the amount of coke adhered to the reforming catalyst in the fluidized bed reactor is insufficient to allow the combustion of the coke inside the regenerator to generate the necessary heat for the reforming reaction (endothermic reaction) of the feedstock inside the fluidized bed reactor. As a result, the feedstock must be preheated in heating furnace to a temperature at least as high as the reaction temperature prior to supply to the fluidized bed reactor.

However, when the feedstock is heated in a heating furnace, some of the feedstock inside the heating furnace exists at the dew point between a mixed gas-liquid state and a completely gaseous phase. Generally, when feedstock at the dew point exists inside the heating furnace, impurities tend to accumulate on the pipes of the heating furnace in the vicinity of the dew point feedstock. These accumulated impurities tend to cause localized heating, increasing the possibility of damage to the heating pipes, and therefore preheating the feedstock in this manner is not ideal. Further, in those cases where rapid progression of the reforming reaction causes the temperature inside the fluidized bed reactor to decrease abruptly, the required heat cannot be supplied rapidly to the system. Accordingly, there are a number of issues that must be addressed in order to enable a fluidized bed reactor to be used for producing a product oil containing BTX in an efficient and stable manner.

### CITATION LIST

### Patent Document

Patent Document 1: Published Japanese Translation of PCT No. H 3-503656

### DISCLOSURE OF INVENTION

### Problems to be solved by the Invention

The present invention provides a method for producing aromatic hydrocarbons using LCO produced from an FCC apparatus, naphtha and straight-run gas oil as the feedstock, which enables the aromatic hydrocarbons to be produced in an efficient and stable manner.

### Means for Solving the Problem

A method for producing aromatic hydrocarbons according to the present invention involves bringing at least one feedstock oil selected from the group consisting of LCO produced from an FCC apparatus, hydrotreated LCO, naphtha and straight-run gas oil into contact with a reforming catalyst inside a fluidized bed reactor, wherein the method includes:
transporting a reforming catalyst that has been extracted from the fluidized bed reactor to a heating tank,
heating the reforming catalyst in the heating tank to a temperature at least as high as the reaction temperature inside the fluidized bed reactor, and
following heating, transporting the heated reforming catalyst to the fluidized bed reactor.

The heating of the reforming catalyst in the heating tank is preferably performed by combusting, in the presence of an oxygen-containing gas, a heating fuel supplied to the heating tank from an external source.
The heating fuel may be either a liquid fuel or a gaseous fuel, and is preferably a product oil distillation tower bottom oil obtained from the production method of the present invention.
The amount of the heating fuel, for example the distillation tower bottom oil, supplied to the heating tank is preferably within a range from 0.005 to 0.08 tons per 1 ton of feedstock oil supplied to the fluidized bed reactor.
The amount of the reforming catalyst extracted from the fluidized bed reactor is preferably within a range from 5 to 30 tons per 1 ton of feedstock oil supplied to the fluidized bed reactor.

The pressure inside the fluidized bed reactor is preferably within a range from 0.1 to 1.5 MPaG
The reaction temperature inside the fluidized bed reactor is preferably within a range from 350 to 700°C.
The contact time between the feedstock oil and the reforming catalyst inside the fluidized bed reactor is preferably within a range from 5 to 300 seconds.

### Advantageous Effects of the Invention

The method for producing aromatic hydrocarbons according to the present invention is a method for producing aromatic hydrocarbons using LCO produced from an FCC apparatus, naphtha and straight-run gas oil as the feedstock, which enables the aromatic hydrocarbons to be produced in an efficient and stable manner.

### BRIEF DESCRIPTION OF THE DRAWINGS

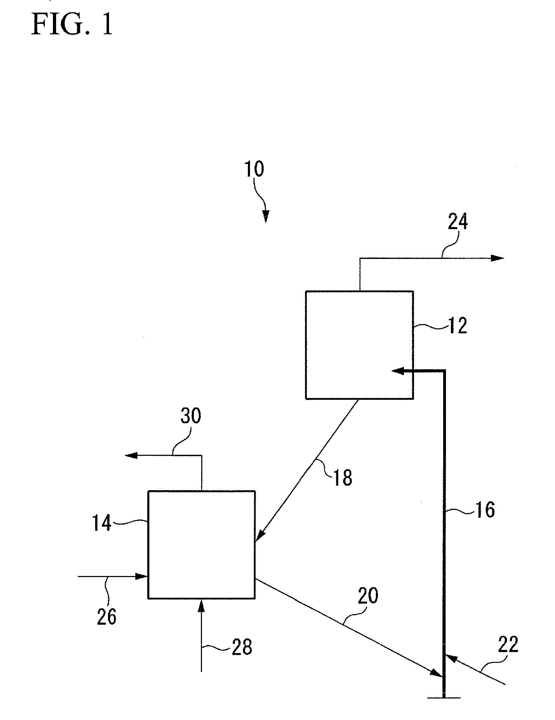

FIG. 1 is a schematic structural diagram illustrating one example of a fluid catalytic reforming apparatus used in the method for producing aromatic hydrocarbons according to the present invention.

### BEST MODE FOR CARRYING OUT THE INVENTION

FIG. 1 is a schematic structural diagram illustrating one example of a fluid catalytic reforming apparatus used in the method for producing aromatic hydrocarbons according to the present invention. A fluid catalytic reforming apparatus 10 includes a fluidized bed reactor 12, a heating tank 14, a catalyst riser 16, an inclined pipe 18, an inclined pipe 20, a feed pipe 22, a discharge pipe 24, a fuel pipe 26, an oxygen-containing gas pipe 28, and an exhaust pipe 30. The terminal of the catalyst riser 16 is connected to the fluidized bed reactor 12. The base of the inclined pipe 18 is connected to the fluidized bed reactor 12, and the terminal of the inclined pipe 18 is connected to the heating tank 14. The base of the inclined pipe 20 is connected to the heating tank 14, and the terminal of the inclined pipe 20 is connected to the base of the catalyst riser 16. The base of the discharge pipe 24 is connected to the fluidized bed reactor 12. The terminal of the fuel pipe 26 is connected to the heating tank 14. The terminal of the oxygen-containing gas pipe 28 is connected to the heating tank 14. The base of the exhaust pipe 30 is connected to the heating tank 14.

The fluidized bed reactor 12 is used for bringing the feedstock oil into contact with the fluidized bed of the reforming catalyst to generate a product oil containing a large proportion of BTX. The fluidized bed reactor 12 includes a supply port, an extraction port, a cyclone, and a discharge port. The feedstock oil vapor and reforming catalyst that have been transported through the catalyst riser 16 are introduced into the fluidized bed reactor 12 via the supply port. The reforming catalyst is extracted into the inclined pipe 18 through the extraction port. The cyclone is used for separating the vapor of the product oil and the reforming catalyst. The vapor of the product oil that has been separated in the cyclone is discharged into the discharge pipe 24 through the discharge port.

The heating tank 14 uses not only the heat generated by combustion of the coke adhered to the reforming catalyst, but also energy supplied from an external source to actively heat the reforming catalyst. In other words, the heating tank 14 is, in itself, a large heating device. The heating tank 14 includes three supply ports, an extraction port and an exhaust port. The reforming catalyst that has been transported through the inclined pipe 18 is introduced into the heating tank 14 through the first supply port. The reforming catalyst is extracted into the inclined pipe 20 through the extraction port. A heating fuel that has been transported from an external source through the fuel pipe 26 is introduced into the heating tank 14 through the second supply port. An oxygen-containing gas that has been supplied through the oxygen-containing gas pipe 28 is introduced into the heating tank 14 through the third supply port. Combustion gases generated by the combustion are discharged into the exhaust pipe 30 through the exhaust port.

The catalyst riser 16 is a pipe-like member that extends in the vertical direction, and includes a supply port via which the reforming catalyst that has been transported through the inclined pipe 20 is introduced, and a supply port via which a liquid feedstock oil that has been supplied through the feed pipe 22 is introduced.

Production of aromatic hydrocarbons using the fluid catalytic reforming apparatus 10 illustrated in FIG. 1 is conducted, for example, in the manner described below.
The feedstock oil, which has been preheated using a preliminary heater (not shown in the figure) provided partway along the feed pipe 22, is introduced continuously from the feed pipe 22 to the catalyst riser 16. At the same time, the reforming catalyst that has been heated in the heating tank 14 is introduced continuously into the catalyst riser 16 from the inclined pipe 20, and is transported into the fluidized bed reactor 12 by the vapor of the vaporized feedstock oil rising up the catalyst riser 16 which acts as a transport medium.

The reforming catalyst that is supplied continuously, together with the vapor of the feedstock oil, from the catalyst riser 16 to the fluidized bed reactor 12 is converted to a fluidized bed state by the vapor of the feedstock oil. The feedstock oil vapor and the reforming catalyst make contact within this fluidized bed state, yielding a product oil vapor that contains a large amount of BTX. The product oil vapor and the reforming catalyst are separated by the cyclone, and the product oil vapor is discharged continuously into the discharge pipe 24. The discharged product oil vapor travels through the discharge pipe 24 and is transported to a distillation tower or the like (not shown in the figure) of a subsequent stage. Coke adheres to the catalyst as a result of the contact with the feedstock oil vapor, and a portion of the partially inactivated reforming catalyst is extracted continuously from the fluidized bed reactor 12 into the inclined pipe 18.

The reforming catalyst that is introduced continuously into the heating tank 14 from the inclined pipe 18 is heated continuously to a temperature at least as high as the reaction temperature inside the fluidized bed reactor 12 by combusting the heating fuel, which has been supplied from an external source through the fuel pipe 26, in the presence of the oxygen-containing gas that has been supplied through the oxygen-containing gas pipe 28. In other words, the heat of combustion generated by combusting the heating fuel and the oxygen-containing gas is used to heat the reforming catalyst. Further, during this heating, the coke adhered to the reforming catalyst also combusts, meaning the reforming catalyst undergoes regeneration during the heating process. The combustion gases generated by the combustion are discharged continuously into the exhaust pipe 30. The heated reforming catalyst is extracted continuously from the heating tank 14 into the inclined pipe 20, and is then re-introduced into the catalyst riser 16 from the inclined pipe 20. In this manner, the reforming catalyst is continuously circulated between the fluidized bed reactor 12 and the heating tank 14.

As the feedstock oil, at least one oil selected from the group consisting of LCO produced from an FCC apparatus, hydrotreated LCO, and naphtha may be used. In those cases when at least one of these feedstock oils is used, the amount of coke that adheres to the reforming catalyst upon contact between the feedstock oil and the reforming catalyst is not necessarily that large. Accordingly, the production method of the present invention is very effective in enabling the efficient and stable production of aromatic hydrocarbons from the feedstock oil.

The reforming catalyst contains a crystalline aluminosilicate.
Although there are no particular limitations on the amount of the crystalline aluminosilicate within the reforming catalyst, the amount is preferably not less than 10% by mass and not more than 95% by mass, more preferably not less than 20% by mass and not more than 80% by mass, and still more preferably not less than 25% by mass and not more than 70% by mass.

Although there are no particular limitations on the crystalline aluminosilicate, examples of preferred aluminosilicates include the medium pore size zeolites such as MFI (Zeolite Socony Mobil-five), MEL (Zeolite Socony Mobil-eleven), TON (Theta-one), MTT (Zeolite Socony Mobil-twenty-three), MRE (Zeolite Socony Mobil-48), FER (Ferrierite), AEL (Aluminophosphate-eleven) and EUO (Edinburgh University-one), and of these, MFI and/or MEL-type crystal structures are particularly desirable. MFI-type and MEL-type aluminosilicates belong to the group of conventional zeolite structures published by The Structure Commission of the International Zeolite Association (Atlas of Zeolite Structure Types, W.M. Meiyer and D.H. Olson (1978), distributed by Polycrystal Book Service, Pittsburgh, PA, USA).

The crystalline aluminosilicate preferably contains gallium and/or zinc. By including gallium and/or zinc, BTX can be produced more efficiently, and the production of non-aromatic hydrocarbon by-products of 3 to 6 carbon atoms can be suppressed significantly.
Examples of crystalline aluminosilicates containing gallium and/or zinc include catalysts in which gallium is incorporated within the lattice framework of the crystalline aluminosilicate (crystalline aluminogallosilicates), catalysts in which zinc is incorporated within the lattice framework of the crystalline aluminosilicate (crystalline aluminozincosilicates), catalysts in which gallium is supported on the crystalline aluminosilicate (gallium-supporting crystalline aluminosilicates), catalysts in which zinc is supported on the crystalline aluminosilicate (zinc-supporting crystalline aluminosilicates), and catalysts including one or more of these forms.

A gallium-supporting crystalline aluminosilicate and/or zinc-supporting crystalline aluminosilicate can obtained by supporting gallium and/or zinc on a crystalline aluminosilicate using a conventional method such as an ion-exchange method or an impregnation method. There are no particular limitations on the gallium source and zinc source used in these methods, and examples include gallium salts such as gallium nitrate and gallium chloride, gallium oxide, zinc salts such as zinc nitrate and zinc chloride, and zinc oxide.

A crystalline aluminogallosilicate and/or crystalline aluminozincosilicate has a structure in which SiO₄, AlO₄ and GaO₄/ZnO₄ structures adopt tetrahedral coordination within the framework. These crystalline aluminogallosilicates and/or crystalline aluminozincosilicates can be obtained by gel crystallization via hydrothermal synthesis, by a method in which gallium and/or zinc is inserted into the lattice framework of a crystalline aluminosilicate, or by a method in which aluminum is inserted into the lattice framework of a crystalline gallosilicate and/or crystalline zincosilicate.

The heating fuel acts as an additional fuel besides the coke adhered to the reforming catalyst, and examples include fuels supplied from externally (so-called torch oil), such as the distillation tower bottom oil from the product oil obtained in the production method of the present invention. In terms of avoiding the problem of degradation of the reforming catalyst caused by water vapor, the heating fuel is preferably a distillation tower bottom oil having a comparatively large ratio of carbon atoms to hydrogen atoms (C/H).
Examples of the oxygen-containing gas include air and pure oxygen, although air is preferred from an economic viewpoint.

The heating of the reforming catalyst is not limited to heating generated by combustion of a heating fuel, and heating may also be performed using an indirect heating device such as a heater or the like. However, from the viewpoints of heating efficiency and suppression of reforming catalyst degradation, heating generated by combustion of a heating fuel is preferred.

Because the heat required by the reforming reaction inside the fluidized bed reactor 12 is supplied by the heated reforming catalyst, the heating temperature of the feedstock oil by the preliminary heater (not shown in the figure) may be any temperature less than the reaction temperature inside the fluidized bed reactor 12. The heating temperature for the feedstock oil is preferably not less than 150°C and not more than 450°C, and is more preferably not less than 180°C and not more than 350°C.

The pressure inside the fluidized bed reactor 12 varies depending on the target reaction yield, but the lower limit for the pressure is preferably 0.1 MPaG, and more preferably 0.2 MPaG. On the other hand, the upper limit for the pressure is preferably 1.5 MPaG, more preferably 1.0 MPaG, and still more preferably 0.5 MPaG. Provided the pressure is at least 0.1 MPaG, BTX can be produced efficiently. Provided the pressure is not more than 1.5 MPaG, the amount of light gas by-products generated by cracking can be suppressed.

The lower limit for the reaction temperature inside the fluidized bed reactor 12 is preferably 350°C, more preferably 450°C, still more preferably 500°C, and still more preferably 520°C. On the other hand, the upper limit for the reaction temperature is preferably 700°C, and more preferably 600°C. Provided the reaction temperature is at least 350°C, the activity of the reforming catalyst reaches a satisfactory level. Provided the reaction temperature is not more than 700°C, excessive cracking reactions can be avoided.

The lower limit for the contact time between the feedstock oil and the reforming catalyst inside the fluidized bed reactor 12 is preferably 5 seconds, more preferably 10 seconds, and still more preferably 15 seconds. On the other hand, the upper limit for the contact time is preferably 300 seconds, more preferably 150 seconds, and still more preferably 100 seconds. Provided the contact time is at least 5 seconds, the reforming reaction progresses satisfactorily. Provided the contact time is not more than 300 seconds, the amount of light gas by-products generated by cracking can be suppressed.

The amount of the reforming catalyst extracted from the fluidized bed reactor 12 (the circulation amount) is preferably within a range from 5 to 30 tons per 1 ton of the feedstock oil supplied to the fluidized bed reactor 12. This amount is also determined in accordance with the overall heat balance.

The pressure inside the heating tank 14 is preferably higher than the pressure inside the fluidized bed reactor 12 in order to facilitate transport of the heated reforming catalyst to the fluidized bed reactor 12.
Because the heat required by the preforming reaction inside the fluidized bed reactor 12 is supplied by the heated reforming catalyst, the temperature inside the heating tank 14 must be at least as high as the reaction temperature inside the fluidized bed reactor 12. The lower limit for the temperature inside the heating tank 14 is preferably 500°C, and more preferably 600°C. On the other hand, the upper limit for the temperature is preferably 800°C, and more preferably 700°C.

The amount of the heating fuel supplied to the heating tank 14 (in the case of a distillation column bottom oil), is preferably not less than 0.005 tons and not more than 0.08 tons, and more preferably not less than 0.02 tons and not more than 0.08 tons, per 1 ton of the feedstock oil supplied to the fluidized bed reactor 12. The amount of the heating fuel supplied is determined in accordance with the amount of coke generated and the overall heat balance.

In the method for producing aromatic hydrocarbons according to the present invention described above, the reforming catalyst extracted from the fluidized bed reactor is transported to the heating tank and heated to a temperature at least as high as the reaction temperature inside the fluidized bed reactor, and the heated reforming catalyst is then transported back into the fluidized bed reactor. Accordingly, by using not only the heat from combustion of the coke adhered to the reforming catalyst, but also the energy supplied from an external source to actively heat the reforming catalyst, the heat required for maintaining the reforming reaction (endothermic reaction) inside the fluidized bed reactor can be supplied in an efficient and stable manner. As a result, even in those cases where a feedstock oil (such as an LCO) is used for which the amount of coke that adheres to the reforming catalyst upon contact with the reforming catalyst is insufficient, BTX can still be produced in an efficient and stable manner.

### EXAMPLES

An example is presented below.

### [Example 1]

Using the fluid catalytic reforming apparatus 10 having the structure illustrated in FIG. 1, BTX production was performed under the operating conditions listed below, and the resulting data was used to calculate the production rate and the like for BTX.

### (Operating conditions)

Heating temperature of feedstock oil by preliminary heater (not shown in the figure): 200°C
Supply rate of the feedstock oil (vapor) to the catalyst riser 16: 1 ton/hour
Pressure inside the fluidized bed reactor 12: 0.3 MPaG
Reaction temperature inside the fluidized bed reactor 12: 560°C
Contact time between the feedstock oil and the reforming catalyst inside the fluidized bed reactor 12: 18 seconds
Pressure inside the heating tank 14: 0.35 MPaG
Temperature inside the heating tank 14: 650°C
Supply rate of the heating fuel to the heating tank 14: 0.025 tons / 1 ton of the feedstock oil
Supply rate of air to the heating tank 14: 0.80 tons / 1 ton of the feedstock oil
Circulation rate of the reforming catalyst: 17.6 tons / 1 ton of the feedstock oil

An LCO was used as the feedstock oil.
The distillation tower bottom oil from the product oil was used as the heating fuel (torch oil).
A reforming catalyst containing an MFI-type zeolite (particle dimensions: approximately 0.3 µm) having gallium incorporated within the lattice framework was used as the reforming catalyst.

During operation, heat was able to be supplied efficiently to the fluidized bed reactor 12 using the reforming catalyst that had been heated in the heating tank 14, and a product oil was able to be obtained in a stable manner, without major fluctuations in the temperature inside the fluidized bed reactor 12.
The amount of BTX contained within the product oil was 43% by mass.

### INDUSTRIAL APPLICABILITY

The present invention is useful for producing aromatic hydrocarbons using LCO produced from an FCC apparatus, and naphtha as a feedstock.

### REFERENCE NUMBER

- 12:: Fluidized bed reactor
- 14:: Heating tank

## Claims

1. A method for producing aromatic hydrocarbons by bringing at least one feedstock oil selected from the group consisting of light cycle oil produced from a fluid catalytic cracking apparatus, hydrotreated light cycle oil, naphtha and straight-run gas oil into contact with a reforming catalyst inside a fluidized bed reactor, wherein the method comprises:
transporting a reforming catalyst that has been extracted from the fluidized bed reactor to a heating tank,
heating the reforming catalyst in the heating tank to a temperature at least as high as a reaction temperature inside the fluidized bed reactor, and
following heating, transporting the heated reforming catalyst to the fluidized bed reactor.

2. The method for producing aromatic hydrocarbons according to claim 1, wherein the heating of the reforming catalyst in the heating tank is performed by combusting, in presence of an oxygen-containing gas, a heating fuel supplied to the heating tank from an external source.

3. The method for producing aromatic hydrocarbons according to claim 2, wherein the heating fuel is a distillation tower bottom oil from the product oil.

4. The method for producing aromatic hydrocarbons according to claim 3, wherein an amount of the distillation tower bottom oil supplied to the heating tank is within a range from 0.005 to 0.08 tons per 1 ton of feedstock oil supplied to the fluidized bed reactor.

5. The method for producing aromatic hydrocarbons according to any one of claims 1 to 4, wherein an amount of the reforming catalyst extracted from the fluidized bed reactor is within a range from 5 to 30 tons per 1 ton of feedstock oil supplied to the fluidized bed reactor.

6. The method for producing aromatic hydrocarbons according to any one of claims 1 to 5, wherein a pressure inside the fluidized bed reactor is within a range from 0.1 to 1.5 MPaG

7. The method for producing aromatic hydrocarbons according to any one of claims 1 to 6, wherein a reaction temperature inside the fluidized bed reactor is within a range from 350 to 700°C.

8. The method for producing aromatic hydrocarbons according to any one of claims 1 to 7, wherein a contact time between the feedstock oil and the reforming catalyst inside the fluidized bed reactor is within a range from 5 to 300 seconds.
